(19) **Europäisches Patentamt / European Patent Office / Office européen des brevets**

(11) **EP 2 630 910 B1**

(12) **EUROPÄISCHE PATENTSCHRIFT**

(45) Veröffentlichungstag und Bekanntmachung des Hinweises auf die Patenterteilung:
**30.07.2014 Patentblatt 2014/31**

(51) Int Cl.:
*A61B 5/0452* ^(2006.01)  *A61B 5/0476* ^(2006.01)
*A61B 5/0496* ^(2006.01)

(21) Anmeldenummer: **13455001.1**

(22) Anmeldetag: **29.01.2013**

(54) **Verfahren zur Detektion von Artefakten**

Method for detecting artefacts

Procédé de détection d'artéfacts

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.02.2012 AT 2372012**

(43) Veröffentlichungstag der Anmeldung:
**28.08.2013 Patentblatt 2013/35**

(73) Patentinhaber: **AIT Austrian Institute of Technology GmbH**
**1220 Wien (AT)**

(72) Erfinder:
• **Hayn, Dieter**
**8010 Graz (AT)**
• **Schreier, Günter**
**8010 Graz (AT)**

(74) Vertreter: **Wildhack & Jellinek**
**Patentanwälte**
**Landstraßer Hauptstraße 50**
**1030 Wien (AT)**

(56) Entgegenhaltungen:
**EP-A1- 0 580 894    EP-A1- 2 407 097**
**US-A1- 2005 033 126**

• **HAYN D ET AL: "ECG quality assessment for patient empowerment in mHealth applications", COMPUTING IN CARDIOLOGY, Bd. 38, 18. September 2011 (2011-09-18), Seiten 353-356, XP032167277, ISBN: 978-1-4577-0612-7**
• **DARRINGTON ET AL: "Towards real time QRS detection: A fast method using minimal pre-processing", BIOMEDICAL SIGNAL PROCESSING AND CONTROL, Bd. 1, Nr. 2, 1. April 2006 (2006-04-01), Seiten 169-176, XP028019073, ISSN: 1746-8094, DOI: 10.1016/J.BSPC.2006.08.002 [gefunden am 2006-04-01]**

**Beschreibung**

Die Erfindung betrifft ein Verfahren zur Detektion von Artefakten in einem Biosignal mit wiederholt auftretenden Ereignissen.

Unter Biosignalen versteht man Signale, die die Werte bestimmter an biologischen Systemen erfasster veränderlicher physikalischer Größen, mit der Zeit angeben. Beispiele für Biosignale sind das Elektrokardiogramms (EKG), das Elektroenzephalogramm (EEG) oder das Elektrookulogramm (EOG). Ein wesentlicher Bestandteil in der Analyse solcher Biosignale ist die Erkennung von bestimmten Ereignissen, wie beispielsweise die Detektion von Signalteilen, die im Falle des EKGs durch die Erregung des Herzens entstanden sind (z.B. der QRS-Komplexe) oder im Falle von EOGs ihren Ursprung im Zwinkern der Augen haben. Viele dieser Ereignisse treten wiederholt in relativ regelmäßigen Abständen auf. Inhalt der gegenständlichen Erfindung ist ein Verfahren zur Detektion derartiger Ereignisse.

Mit dem beschriebenen Verfahren werden Artefakte in einem Biosignal mit wiederholt auftretenden Ereignissen detektiert, indem das Biosignal in eine Anzahl von Teilsignalen unterteilt wird, und für jedes einzelne Teilsignal nach einer Anzahl von Ereignissen gesucht wird, deren Amplitude den größten Wert aufweist. Die so aufgefundenen Ereignisse werden nach ihrer Amplitude sortiert, und die Amplitude des Ereignisses mit der kleinsten verbleibende Amplitude sowie die nächst kleinere Amplitude des in der Sortierung nachfolgenden Ereignisses als größte verworfene Amplitude werden ermittelt. Zum Erkennen von Artefakten innerhalb eines Teilsignals werden dabei die kleinste verbleibende Amplitude und die größte verworfene Amplitude eines Teilsignals mit den kleinsten verbleibenden Amplituden und mit den größten verworfenen Amplituden der übrigen Teilsignale verglichen, um festzustellen, ob das Teilsignal Artefakte aufweist

Ein Maß für die Bestimmung der Qualität bei der Detektion von Ereignissen ist die Sensitivität. Diese ist festgelegt durch die Anzahl der richtigerweise detektierten Ereignisse dividiert durch die Anzahl aller tatsächlich vorhandenen Ereignisse in einem Signal. Ein weiteres Maß für die Bestimmung der Qualität der Detektion von Ereignissen ist das sogenannte "positive predictive value" (PPV), das durch das Verhältnis der Anzahl der richtig detektierten Ereignisse zur Anzahl aller detektierten Ereignisse festgelegt ist.

Detektoren für wiederholt in relativ regelmäßigen Abständen auftretende Ereignisse werden unter anderem in Geräten zur Aufzeichnung von EKGs verwendet. Problematisch ist dabei, dass die Ausprägung der Ereignisse oft unterschiedlich ist, weshalb im Stand der Technik adaptive Schwellwerte, die sich an das jeweilige Biosignal anpassen, verwendet werden. Die adaptive Bestimmung eines optimalen Schwellenwerts ist jedoch schwierig, für die Sensitivität und PPV einer Detektion kann niemals der Idealfall von 100% erreicht werden.

Ein weiteres Problem liegt darin, dass innerhalb von Biosignalen häufig Signalbereiche mit hohem Störanteil, d. h. mit Artefakten, vorkommen, deren Ursache z. B. in verändertem Elektrodenkontakt durch Bewegung, in Signalen von Skelettmuskeln oder in von extern eingespeisten Störsignalen (50 Hz) liegen können.

In der Patentschrift AT 504167 ist ein Verfahren zur Detektion wiederholt in relativ regelmäßigen Abständen auftretender Ereignisse aus Biosignalen beschrieben, auf welches die vorliegende Erfindung aufbaut. Es werden Ereignisse dadurch detektiert, dass innerhalb des Signals wiederholt der Zeitpunkt und die Amplitude des maximalen Amplitudenwertes des Signals als Ereignis gespeichert wird und das Signal in einem vordefinierten Teilsignal um diesen Zeitpunkt mit einer Unterdrückungsfunktion multipliziert wird, solange, bis die Amplitude des verbleibenden Maximums einen vordefinierten Schwellwerte unterschreitet oder bis die Anzahl der gefundenen Ereignisse eine vordefinierte Schranke überschreitet und innerhalb der absteigend sortierten Amplituden aller gefundenen Ereignisse, beginnend beim n-ten Ereignis, wobei n der Mindestanzahl der zu detektierenden Ereignisse entspricht, nach dem deutlichsten Sprung gesucht wird und alle Ereignisse mit Amplituden, die hinter diesem Sprung liegen, verworfen werden.

Nach der Detektion kann ein Qualitätsmaß bestimmt werden, das angibt, wie zuverlässig die jeweilige Detektion war, indem beispielsweise die Höhe des gefundenen Sprungs bestimmt wird.

Problematisch bei diesem Verfahren ist, dass auftretende Artefakte häufig deutlich höhere Amplituden aufweisen als das Signal selbst. Dadurch kann auch der deutlichste Sprung innerhalb der Amplituden möglicher Ereignisse falsch erkannt werden - nämlich zwischen der minimalen Artefaktamplitude und der maximalen Ereignis-Amplitude anstatt zwischen minimaler Ereignis-Amplitude und maximaler verworfener Amplitude. Andererseits kann im Fall fehlender Signal-Anteile, beispielsweise Kurzschluss von Elektroden, der Sprung innerhalb von Rausch-Anteilen des Signals gefunden werden.

Es ist auch bekannt, dass das Signal vor der beschriebenen Ereignisdetektion in einzelne Zeitfenster bzw. Teilsignale unterteilt werden kann und die Detektion für jedes Teilsignal getrennt durchgeführt wird. Dadurch kann - auch im Fall von Artefakten - die Detektion in allen artefaktfreien Teilsignalen erfolgreich durchgeführt werden. Lediglich in Teilsignalen mit schlechter Signalqualität würden zu viele oder zu wenig Ereignisse detektiert werden. Dennoch ist die Genauigkeit dieses Verfahrens - insbesondere in den Teilsignalen mit schlechter Signalqualität - in vielen Fällen nicht zufriedenstellend, denn in diesen Teilsignalen werden Artefakte oder Rauschanteile als Ereignisse interpretiert, wodurch die Spezifität sinkt, und tatsächliche Ereignisse werden dafür nicht erkannt, wodurch die Sensitivität ebenfalls sinkt.

Der Erfindung liegt demnach die Aufgabe zugrunde, wiederholt in relativ regelmäßigen Abständen auftretende Ereignisse innerhalb von Biosignalen mit maximaler Sensitivität und Spezifität zu detektieren und diese Ereignisse optimal von Artefakten zu unterscheiden.

Die Erfindung löst diese Aufgabe bei einem Verfahren der eingangs genannten Art mit den kennzeichnenden Merkmalen des Patentanspruchs 1.

Erfindungsgemäß ist bei einem Verfahren zur Detektion von Artefakten in einem Biosignal mit wiederholt auftretenden Ereignissen,

wobei das Biosignal in eine Anzahl von Teilsignalen, insbesondere derselben Länge, unterteilt wird, und für jedes einzelne Teilsignal

- nach einer Anzahl von Ereignissen gesucht wird, und die Amplitude des Teilsignals zum Zeitpunkt des Auftretens des Ereignisses ermittelt wird,
- die so aufgefundenen Ereignisse nach ihrer Amplitude sortiert werden, und
- nach vorgegebenen Kriterien die Amplitude eines Ereignisses als kleinste verbleibende Amplitude ermittelt und ausgewählt wird und die nächst kleinere Amplitude des in der Sortierung nachfolgenden Ereignisses als größte verworfene Amplitude ermittelt wird, vorgesehen, dass

zum Erkennen von Artefakten innerhalb eines Teilsignals

die kleinste verbleibende Amplitude eines Teilsignals und/oder die größte verworfene Amplitude eines Teilsignals mit den kleinsten verbleibenden Amplituden der übrigen Teilsignale und/oder mit den größten verworfenen Amplituden der übrigen Teilsignale verglichen wird und

dass bei Vorliegen von durch diesen Vergleich ermittelten, einen vorgegebenen Schwellenwert überschreitenden Abweichungen festgestellt wird, dass das Teilsignal Artefakte aufweist. Ein bevorzugter Aspekt der Erfindung sieht vor, dass zur Bestimmung der kleinsten verbleibenden Amplitude eines Teilsignals nach einem Ereignis gesucht wird, dessen Amplitude zur jeweils nächst kleineren Amplitude unter allen Ereignissen den größten absoluten oder relativen Abstand hat, und diese Amplitude als kleinste verbleibende Amplitude dem Teilsignal zugeordnet wird und die nächstkleinere Amplitude dem Teilsignal als größte verworfene Amplitude zugeordnet wird.

Beispielsweise werden alle detektierten Ereignisse nach ihrem Wert sortiert und innerhalb der sortierten Amplituden wird nach dem größten relativen oder absoluten Abstand oder Sprung gesucht. Alle Ereignisse vor dem Sprung mit einer Amplitude größer als die minimale verbleibende Amplitude $amp_{E,min}$ werden behalten und die übrigen mit einer Amplitude kleiner als die maximale verworfene Amplitude $amp_{R,max}$ werden verworfen.

Dieses Vorgehen ermöglicht es, gültige Ereignisse zu detektieren, indem entschieden wird, ab welcher Amplitude Ereignisse als gültig interpretiert werden.

Zur Beschleunigung des Verfahrens kann vorgesehen sein, dass zur Bestimmung der kleinsten verbleibenden Amplitude eines Teilsignals

- für eine Anzahl von unmittelbar vorangehenden Teilsignalen ein Schwellenwert als, insbesondere arithmetischer, Mittelwert aller kleinsten verbleibenden Amplituden und aller größten verworfenen Amplituden mit oder ohne Gewichtung ermittelt wird,
- im jeweiligen Teilsignal das Ereignis mit der kleinsten diesen Schwellenwert übersteigenden Amplitude ausgewählt und diese Amplitude als kleinste verbleibende Amplitude für dieses Teilsignal ermittelt wird, und
- die jeweils nächstkleinere Amplitude als größte verworfene Amplitude für dieses Teilsignal ermittelt wird.

Zur Vereinfachung der Berechnung sowie zur Beschleunigung des Verfahrens kann vorgesehen sein, dass zur Bestimmung der kleinsten verbleibenden Amplitude eines Teilsignals nach einer vorgegebenen Anzahl von Ereignissen gesucht wird, deren Amplitude sich am stärksten von der nächstkleineren Amplitude unterscheidet, und dass unter diesen Ereignissen und ihren zugehörigen Amplituden diejenige Amplitude als kleinste verbleibende Amplitude des Teilsignals ausgewählt wird, bei der ein aus dieser Amplitude und der nächstkleineren Amplitude nach vorgegebenen Vorgaben berechneter Wert, insbesondere deren Mittelwert, oder die Amplitude selbst oder die nächstkleinere Amplitude selbst, am nächsten bei einem vorgegebenen Schwellenwert, insbesondere beim Mittelwert der nach denselben Vorgaben jeweils in den übrigen Teilsignalen berechneten Werte, liegt.

Zur Bestimmung der Qualität des jeweils ermittelten Teilsignals kann vorgesehen sein, dass ein Qualitätsmaß für jedes Teilsignal ermittelt wird, das eine oder mehrere der folgenden Eigenschaften aufweist:

a) der Unterschied zwischen der kleinsten verbleibenden Amplitude und der größten verworfenen Amplitude ist proportional zum Qualitätsmaß, wobei ein großer Unterschied eine gute Qualität des Teilsignals indiziert,
b) treten die gültigen Ereignisse in regelmäßigen Abständen auf, so indiziert dies eine gute Qualität des Teilsignals,
c) liegen die Amplituden der gültigen Ereignisse in einem vorgegebenen physiologisch erwartbaren Bereich auf, so indiziert dies eine gute Qualität des Teilsignals,

d) ähneln die kleinesten verbleibenden Amplituden und/oder die größten verworfenen Amplituden im jeweiligen Teilsignal jenen einer Anzahl anderer Teilsignale desselben Biosignals, so indiziert das eine gute Qualität des Teilsignals.

Zu diesem Zweck kann insbesondere vorgesehen sein, dass das Qualitätsmaß als gewichtete Summe oder als Produkt eines oder mehrerer der folgenden Qualitätsteilmaße ermittelt wird:

a) Q1 = (kleinste verbleibende Amplitude - größte verworfene Amplitude) / größte verbleibende Amplitude,
b) Q2 = 1 / (Standardabweichung der zeitlichen Abstände aller aufeinanderfolgenden gültigen Ereignisse),
c) Q3 = 1, falls die Amplituden aller gültigen Ereignisse zwischen 0.1 und 3 mV liegen, sonst 0.1,
d) Q4 = 1 / (abs $(amp_{R,max}$ - mean$(amp_{R,max}$ (t)) + abs$(amp_{E,min}$ - mean $(amp_{E,min}$ (t)),

wobei das Qualitätsmaß insbesondere gemäß der folgenden Formel ermittelt wird:

$$Q = Q1 * Q2 * Q3 * Q4 \qquad \text{oder} \qquad Q = Q1 + Q2 + Q3 + Q4$$

Alternativ kann zur Unterscheidung gültiger und ungültiger detektierter Ereignisse vorgesehen sein, dass zur Bestimmung der kleinsten verbleibenden Amplitude innerhalb des Teilsignals für eine Anzahl von Ereignissen, insbesondere für alle Ereignisse, das Qualitätsmaß unter der Annahme ermittelt wird, dass die dem jeweiligen Ereignis zugehörende Amplitude die kleinste verbleibende Amplitude ist, und die kleinste verbleibende Amplitude als demjenigen Ereignis zugeordnete Amplitude bestimmt und festgelegt wird, für das das jeweilige Teilsignal einen Qualitätswert erzielt, der unter allen Ereignissen die höchste Qualität indiziert.

Ein Vorgehen, das einerseits eine rasche und sichere Bestimmung gültiger Ereignisse ermöglicht, sieht vor,

- dass die Bestimmung der kleinsten verbleibenden Amplitude eines Teilsignals nach einem der Ansprüche 3 oder 4 erfolgt,
- dass für dieses Teilsignal jeweils ein Qualitätswert nach einem der Ansprüche 5 oder 6 ermittelt wird, und
- falls der Qualitätswert eines Teilsignals eine schlechtere Qualität indiziert als ein vorgegebener Schwellenwert oder sich die Qualität um mehr als einen vordefinierten Schwellwert von jener einer vorgegebenen Auswahl anderer Teilsignale unterscheidet, die kleinste verbleibende Amplitude eines Teilsignals gemäß einem Verfahren nach einem der Ansprüche 2 oder 7 bestimmt wird.

Zur Detektion eines Artefakts kann vorgesehen sein, dass Teilsignale dann als mit Artefakten behaftet angesehen werden, wenn in diesen Teilsignalen entweder die minimale verbleibende Amplitude kleiner ist als das Maximum der bisher ermittelten, und gegebenenfalls gespeicherten, maximalen verworfenen Amplituden oder die maximale verworfene Amplitude größer ist als das Minimum der bisher ermittelten, und gegebenenfalls gespeicherten, minimalen verbleibenden Amplituden.

Zur Erstellung eines von Artefakten freien Signals kann vorgesehen sein, dass Teilsignale verworfen und nicht zur Beurteilung nachfolgender Teilsignale herangezogen, und insbesondere nicht abgespeichert, werden, wenn ihr Qualitätsmaß einen vorgegebenen Schwellenwert unterschreitet oder wenn sie als mit Artefakten behaftet detektiert worden sind.

Eine vorteilhafte Auswahl von Teilsignalen im Biosignal, die eine vollständige Beurteilung des Biosignals zulässt, sieht vor, dass die Teilsignale im Biosignal lückenlos und unmittelbar hintereinander liegen oder die Teilsignale einander überlappen.

Zur einfachen Handhabung sieht eine weitere vorteilhafte Auswahl von Teilsignalen im Biosignal alternativ oder zusätzlich vor, dass die Teilsignale gleich lang sind und/oder die Startzeitpunkte aufeinanderfolgender oder überlappender Teilsignale zeitlich gleich weit voneinander beabstandet sind.

Ein Vorgehen, das eine verbesserte Detektionsgenauigkeit sowie eine verbesserte Anpassung an konkrete Biosignale ermöglicht, sieht vor, dass, insbesondere zur Beschleunigung des Verfahrens, bei der Beurteilung eines Biosignals mit zeitlich überlappenden Teilsignalen die bereits berechneten Werte überlappender Teilsignale zwischengespeichert werden und jeweils nur Werte für die hinzu gekommenen Zeitbereiche erneut berechnet und die im neuen Teilsignal nicht mehr vorhandenen Zeitbereiche verworfen werden.

Zur weiteren Beschleunigung des Verfahrens kann vorgesehen sein, dass, insbesondere zur Beschleunigung des Verfahrens, innerhalb des aktuellen Teilsignals nur Amplituden als größte verworfene Amplitude in Betracht gezogen werden, die kleiner sind als ein aus einer Anzahl kleinster verbleibender Amplituden anderer Teilsignale berechneter Wert, insbesondere als deren Maximum oder deren Mittelwert, und/oder nur Amplituden als kleinste verbleibende Amplitude in Betracht gezogen werden, die größer sind als ein aus einer Anzahl größter verworfener Amplituden anderer Teilsignale berechneter Wert, insbesondere als deren Minimum oder deren Mittelwert.

Ein bevorzugtes Ausführungsbeispiel der Erfindung wird anhand der folgenden Zeichnungen näher dargestellt.

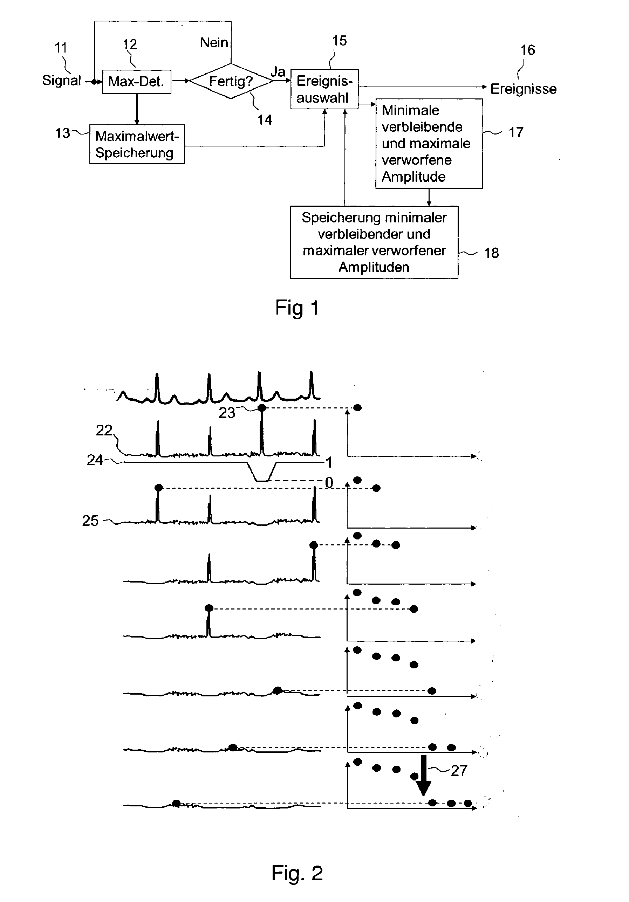

**Fig. 1** zeigt schematisch den Ablauf des Verfahrens gemäß dem bevorzugten Ausführungsbeispiel der Erfindung.

**Fig**. 2 zeigt die Detektion einzelner Ereignisse in einem Teilsignal eines zu untersuchenden Biosignals.

**Fig**. 3 zeigt die abfallende Folge der Amplituden der ermittelten Ereignisse.

**Fig**. 4 zeigt die Unterteilung eines Biosignals in eine Vielzahl von Teilsignale sowie die für die einzelnen Teilsignale ermittelten Folgend von Amplituden der Ereignisse.

**Fig**. 5 zeigt den Verlauf der kleinsten verbleibenden Amplituden sowie den Verlauf der größten verworfenen Amplituden innerhalb der Teilsignale aufgetragen über den jeweiligen Anfangszeitpunkt des Teilsignals.

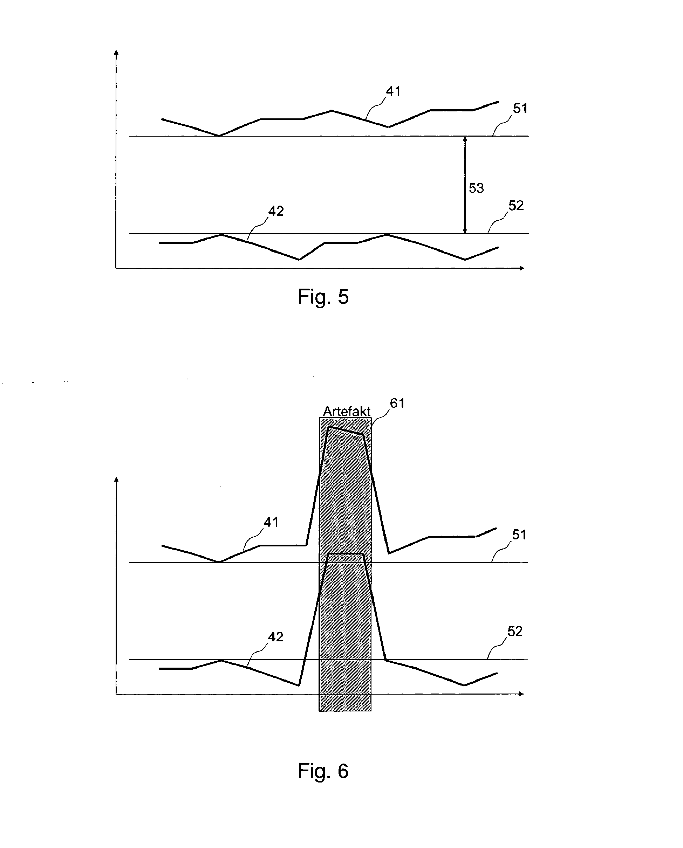

**Fig**. 6 zeigt den in **Fig. 5** dargestellten Signalverlauf bei einem mit Artefakten behafteten Biosignal.

**Fig. 7** zeigt schematisch eine Methode zur Einschränkung der in Frage kommenden Werte für die größte verworfene Amplituden sowie die kleinste verbleibende Amplitude.

**Fig. 8** zeigt eine weitere beschleunigte Detektion der größten verworfenen Amplitude sowie der kleinsten verbleibenden Amplitude.

Mithilfe von nicht dargestellten Sensoren werden bei einem biologischen System, im vorliegenden Fall handelt es sich um das Herz eines Menschen, elektrische Größen an der Hautoberfläche mittels EKG-Elektroden bestimmt. Das Biosignal 1 ist somit im vorliegenden Fall ein EKG-Signal. Alternativ kann auch ein anderes Biosignal, beispielsweise auch ein EEG-Signal oder ein EOG-Signal, herangezogen werden.

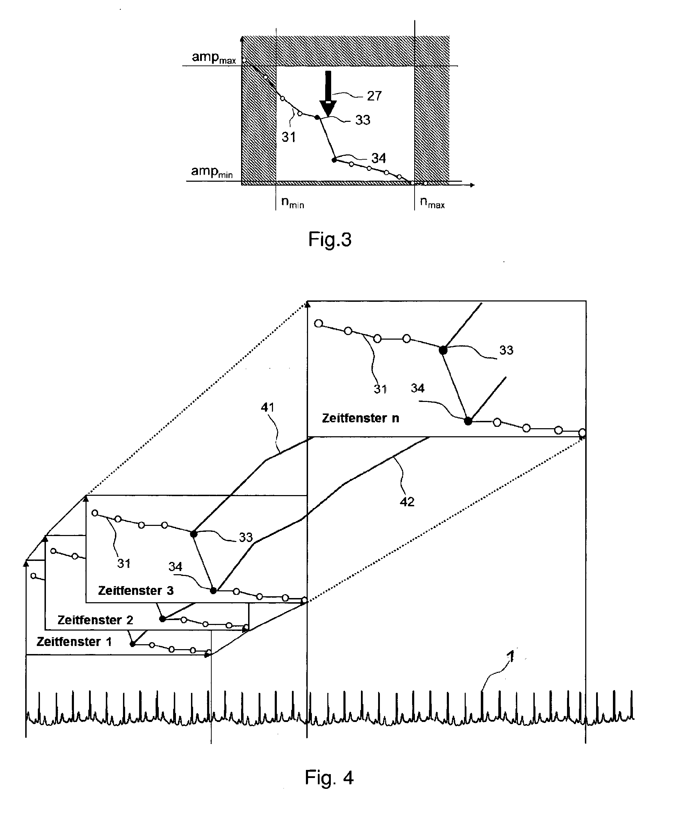

Typisch bei dieser Art von Biosignal 1 (**Fig. 4**) ist, dass bestimmte Ereignisse wie die Erhöhung einer Signalamplitude wiederholt in relativ regelmäßigen Abständen auftritt. Bei der Analyse solcher Biosignale 1 werden diese Ereignisse detektiert, wie beispielsweise die Detektion von Signalteilen, die im Falle des EKGs durch die Erregung des Herzens entstanden sind, beispielsweise der QRS-Komplexe, oder im Falle von EOGs ihren Ursprung im Zwinkern der Augen haben.

**Fig. 1** zeigt ein Blockdiagramm einer Ausführungsform des Verfahrens. Das gemessene Biosignal 1 wir in eine Anzahl von Teilsignalen 11 derselben Länge unterteilt (**Fig. 4**), die einzelnen Teilsignale 11 werden einer Maximum-Detektionseinheit 12 zugeführt. Für das jeweilige Teilsignal 11 werden Ereignisse detektiert. In dieser besonderen Ausführungsform der Erfindung wird das jeweilige Teilsignal 11 dazu vorher gefiltert und es wird der Absolutbetrag gebildet.

Wie in **Fig. 2** dargestellt, wird der maximale Wert innerhalb des Teilsignals 11 bzw. 22 ermittelt und als mögliches Ereignis im Ereignisspeicher 13 abgespeichert. Hierbei werden die Amplitude und der Zeitpunkt des Auftretens dieses maximalen Werts einander zugeordnet und abgespeichert. Um eine erneute Detektion dieses Ereignisses zu vermeiden, wird das Teilsignal 22 mit einer Auslösch-Funktion 24 multipliziert.

Die Auslösch-Funktion 24 weist im Bereich des Zeitpunkts des maximalen Werts bzw. des Ereignisses 23 den Wert Null auf, mit zunehmender Entfernung vom Zeitpunkt des maximalen Werts weist die Auslösch-Funktion 24 den Wert Eins auf oder nähert sich diesem asymptotisch an. Im vorliegenden Fall weist die Auslösch-Funktion 24 in einem vorgegebenen Zeitbereich von +/- 150 ms rund um den maximalen Wert bzw. um das Ereignis 23 den Wert Null auf, in einem vorgegebenen Zeitbereich von mehr als +/- 300 ms weist die Auslösch-Funktion 24 einen Wert von Eins auf. Im Zwischenbereich, d.h. steigt die Auslösch-Funktion 24 linear an oder sie fällt linear ab.

Nach der Anwendung der Auslösch-Funktion 24 auf das jeweilige Teilsignal 22 erhält man ein neues Teilsignal 25, welches das Ereignis 23 mit dem maximalen Wert nicht mehr aufweist. Auf dieses neue Teilsignal 25 wird das vorstehend beschriebene Vorgehen erneut angewandt, es wird erneut ein der maximaler Wert innerhalb des neuen Teilsignals 25 ermittelt und gemeinsam mit seinem zugeordneten Zeitpunkt im Ereignisspeicher 13 abgespeichert. Das detektierte Ereignis 23 wird anschließend durch Multiplikation mit einer Auslösch-Funktion 24 ausgelöscht.

Dieses Vorgehen wird so lange wiederholt, bis eine bestimmte Anzahl $n_{max}$ von Ereignissen detektiert worden ist oder wenn das gesamte Teilsignal 11 durch die Anwendung der Auslösch-Funktion 24 insgesamt gleich Null ist. Nach der Bestimmung des jeweiligen Ereignisses 23 wird eine diesbezügliche Überprüfung 14 durchgeführt, bei der entschieden wird, ob nach weiteren Ereignissen 23 gesucht werden soll oder ob bereits genügend Ereignisse 23 gefunden wurden und die Suche nach neuen Ereignissen 23 abgebrochen wird. Da jeweils nach dem Ereignis 23 mit der größten Amplitude gesucht wird und dieses Ereignis anschließend aus dem Teilsignal 11 ausgelöscht wird, werden die Ereignisse jeweils sortiert nach ihrer Größe abgespeichert und liegen somit nach ihrer Größe sortiert im Ereignisspeicher 13 vor.

Im vorliegenden Fall werden für ein Teilsignal 11 mit einer Aufnahmelänge von 60 Sekunden 200 Ereignisse 23 mit ihrem jeweils zugeordneten maximalen Wert sowie dem jeweiligen Zeitpunkt ihres Auftretens im Ereignisspeicher 13 abgespeichert. Die Anzahl $n_{max}$ der zu detektierenden Ereignisse 23 entspricht der Anzahl von gültigen Ereignissen 16, die in einem typischen Signal der Signaldauer des jeweiligen Teilsignals 11 maximal auftritt. Bei einem EKG-Signal wird angenommen, dass höchstens 200 Herzschläge pro 1 Minute Signallänge auftreten.

In einem weiteren, in **Fig. 3** dargestellten, Auswahlschritt 15 wird entschieden, welche der detektierten Ereignisse 23 als gültige Ereignisse 16 detektiert und behalten werden, die übrigen so detektierten Ereignisse 23 werden verworfen. Sämtliche Ereignisse 23 liegen, wie bereits erwähnt, im Ereignisspeicher 13 sortiert nach Ihrer jeweiligen Amplitude vor. Es liegt somit eine monoton fallende Folge von einzelnen Amplitudenwerten im Ereignisspeicher 13 vor, wobei jeder Amplitudenwert jeweils größer ist als der ihm nachfolgende Amplitudenwert. Zwischen je zwei aufeinander folgenden Amplitudenwerten wird jeweils der relative Abstand bzw. die Differenz bestimmt, anschließend wird die größte relative Abstand 27 zwischen allen aufeinander folgenden Amplitudenwerten bestimmt.

Das Ereignis 23, dessen Amplitude zur jeweils nächst kleineren Amplitude unter allen Ereignissen 23 den größten absoluten oder relativen Abstand hat, wird als kleinste verbleibende Amplitude 33 dem Teilsignal 11 zugeordnet. Die jeweils nächstkleinere Amplitude wird dem Teilsignal 11 als größte verworfene Amplitude 34 zugeordnet. Die kleinste verbleibende Amplitude 33 und die größte verworfene Amplitude 34 werden in einem dem jeweiligen Teilsignal 11 zugeordneten Speicher 18 abgespeichert. Das Ereignis mit der größten verworfenen Amplitude 33 sowie alle Ereignisse mit kleineren Amplituden werden verworfen. Übrig bleiben die gültigen Ereignisse 16.

Der in **Fig. 3** dargestellte schraffierte Bereich umfasst eine Anzahl von Ereignissen 23, die bei der Ermittlung des größten relativen Abstands nicht herangezogen werden, da hierbei entweder zu viele oder zu wenige Ereignisse 23 als gültige Ereignisse 16 detektiert würden. Bei der Bestimmung der kleinsten verbleibenden Amplitude eines Teilsignals wird ein Ereignis 23 dann verworfen, wenn die Amplitude des Ereignisses 23 kleiner als ein vorgegebener Schwellwert, beispielsweise $amp_{min} = 0.05$ mV ist. Weiters wird bei der Bestimmung der kleinsten verbleibenden Amplitude 33 eines Teilsignals 11 ein Ereignis 23 dann verworfen, wenn seine Amplitude kleiner ist als 20% der maximalen Amplitude innerhalb des Teilsignals 11. Außerdem kann im vorliegenden Beispiel die größte verworfene Amplitude 34 nicht größer als ein weiterer Schwellwert, beispielsweise $amp_{max} = 1.0$ mV sein.

In **Fig. 4** ist das Biosignal 1 sowie die jeweiligen Abschnitte, aus denen die Teilsignale 11 erstellt wurden, dargestellt. Die Bestimmung der kleinsten verbleibenden Amplitude 33 sowie der größten verworfenen Amplitude 34 wird nach dem vorstehend dargestellten Vorgehen durchgeführt, wobei für jedes Teilsignal 11 jeweils die kleinste verbleibende Amplitude 33 sowie der größte verworfene Amplitude 34 zur Verfügung stehen. Im vorliegenden Fall sind sämtliche Teilsignale 11 gleich lang. Der Beginn zweier aufeinanderfolgender Teilsignale 11 ist um jeweils 2 Sekunden gegeneinander verschoben, die Länge der Teilsignale 11 beträgt im vorliegenden Fall etwa 60 Sekunden. Die einzelnen Teilsignale 11 weisen somit große Überlappungen auf.

Durch dieses Vorgehen werden Fehldetektionen auf artefaktbehaftete Teilsignale 11 beschränkt und die Sensitivität und Spezifität direkt vor und direkt nach den Artefakten hoch gehalten. Auf diese Art und Weise werden für jedes Teilsignal 11, beispielsweise viele mögliche Ereignisse 23, Qualitätsmaße Q, minimale verbleibende Amplituden 33 und maximale verworfene Amplituden 34 bestimmt.

Von allen für ein Teilsignal 11 gefundenen Werten kann jetzt ausgewählt werden, welche die am ehesten korrekten sind. Dazu können entweder jene Werte herangezogen werden, bei denen das Qualitätsmaß Q ein Maximum war, oder auch nur Werte, die zu ähnlichen Ergebnissen führten wie in anderen Teilsignalen 11.

Die einzelnen Werte für die jeweils die kleinste verbleibende Amplitude 33 sowie der größte verworfene Amplitude 34 werden anschließend als Funktionen 41, 42 der Zeit dargestellt, wobei jeder kleinsten verbleibenden Amplitude 33 sowie jeder größten verworfenen Amplitude 34 jeweils der Beginn des jeweiligen Teilsignals 11 als zugehöriger Zeitpunkt zugewiesen wird. Die erste Funktion 42 weist jeweils die kleinsten verbleibenden Amplitude 33 der einzelnen Teilsignale 11 auf. Die zweite Funktion 42 weist jeweils die größten verworfenen Amplitude 34 der einzelnen Teilsignale 11 auf.

In **Fig. 5** sind zwei Funktionen 41, 42 näher dargestellt, die basierend auf einem Biosignal 1 erstellt wurden, das frei von Artefakten ist. Das Minimum 51 der ersten Funktion 41, $amp_{E,min}(t)$ und das Maximum 52 der zweiten Funktion 42, $amp_{R,max}(t)$ sind ebenfalls dargestellt. Das Minimum 51 der ersten Funktion 41 ist deutlich größer als das Maximum der zweiten Funktion 42, es besteht ein verhältnismäßig großer Abstand 53 zwischen dem Minimum 51 der ersten Funktion 41 und dem Maximum 52 der zweiten Funktion 42.

Wesentlich anders stellt sich die Situation in **Fig. 6** dar, bei der in der Mitte des Biosignals 11 ein Artefakt vorhanden ist. Durch den Artefakt entsteht im illustrierten Beispiel in zwei Teilsignalen 11, deren Beginn jeweils im Zeitbereich 61 liegt, ein Sprung in beiden Funktionen 41, 42, wobei die jeweiligen Werte der zweiten Funktion 42 das Minimum 51 der ersten Funktion 41 übersteigen. Es ist somit zu erkennen, dass die zweite Funktion 42, $amp_{R,max}(t)$ in diesem Zeitbereich 61 Werte annimmt, die höher als das Minimum 51 der Funktion 41, $amp_{E,min}(t)$ sind. Teilsignale 11, in denen dieses Verhalten auftritt, werden als mit Artefakten behaftet interpretiert und entsprechend markiert. Bei der nachfolgenden Behandlung dieses Verfahrens kann darauf entsprechend reagiert werden.

Im vorliegenden Ausführungsbeispiel wurden die jeweiligen Werte der zweiten Funktion 42, die im Zeitbereich 61 liegen, nicht für die Bildung des Maximums 52 der Funktion 42 mit berücksichtigt.

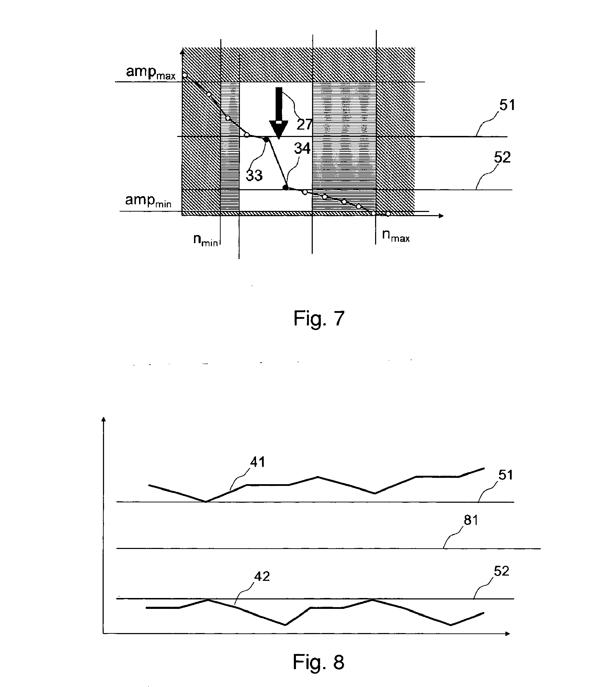

**Fig. 7** zeigt eine weitere Verbesserung des vorstehend dargestellten Verfahrens. Hierbei wird davon ausgegangen, dass das vorstehend genannte Verfahren bereits für eine Anzahl von Teilsignalen 11 des Biosignals 1 durchgeführt wurde und für jedes der Teilsignale 11 jeweils die kleinste verbleibende Amplitude und die größte verworfene Amplitude zur Verfügung stehen. Ferner steht das Minimum 51 der bereits ermittelten kleinsten verbleibenden Amplituden sowie das Maximum 52 der größten verworfenen Amplituden zur Verfügung.

Wie in **Fig. 7** dargestellt, werden lediglich diejenigen Ereignisse 23 ausgewählt, deren jeweilige Amplitude zwischen dem so ermittelten Minimum 51 und dem so ermittelten Maximum 52 liegt; alle Amplituden, die größer als das Minimum 51 sind, werden ohne weitere Berechnungsschritte als gültiges Ereignis 16 interpretiert, alle Amplituden, die kleiner als das Maximum 51 sind, werden verworfen. Das Minimum 51 und das Maximum 52 bilden somit Schwellenwerte 51, 52, nur Ereignisse 23 mit zwischen diesen Schwellenwerten 51, 52 liegenden Amplituden müssen noch als gültiges Ereignis 16 klassifiziert oder verworfen werden. Dazu werden diese Ereignisse 23 sowie das jeweils nächstkleinere und das jeweils nächstgrößere Ereignis 23 näher betrachtet. In **Fig. 7** sind diese näher betrachteten Ereignisse dargestellt, die übrigen Ereignisse 23 befinden sich innerhalb der waagrecht schraffierten Bereiche. Unter den näher betrachteten Ereignissen werden diejenigen zwei benachbarten Ereignisse 33 und 34 gesucht, deren Amplituden zueinander den größten Abstand 27, d. h. die größte Differenz, aufweisen. Von diesen beiden Ereignissen 33 und 34 wird dasjenige mit der größeren Amplitude herangezogen und dessen größere Amplitude gilt als kleinste verbleibende Amplitude 33 des jeweiligen Teilsignals 11. Die jeweils kleinere Amplitude gilt als größte verworfene Amplitude 34.

Eine weitere alternative Vereinfachung und Beschleunigung des Verfahrens ist in **Fig. 8** dargestellt. Wiederum wird davon ausgegangen, dass das mit den **Fig. 1 bis 5** beschriebene Verfahren bereits mehrfach für eine Anzahl von Teilsignalen 11 des Biosignals 1 durchgeführt wurde und für jedes der Teilsignale 11 jeweils die kleinste verbleibende Amplitude 33 und die größte verworfene Amplitude 34 zur Verfügung stehen. Wiederum steht das Minimum 51 der bereits ermittelten kleinsten verbleibenden Amplituden 33 sowie das Maximum 52 der größten verworfenen Amplituden 34 zur Verfügung.

Hierbei kann für eine Anzahl von unmittelbar vorangehenden Teilsignalen der Schwellenwert 81 als, insbesondere arithmetische, Mittelwert aller kleinsten verbleibenden Amplituden 33 und aller größten verworfenen Amplituden 34 mit oder ohne Gewichtung ermittelt werden.

Alternativ kann der jeweilige Schwellenwert 81 als Mittelwert zwischen dem Minimum 51 und dem Maximum 52 gebildet werden. Diejenige Amplitude eines Ereignisses 23, die unter den Amplituden, die größer als der Schwellenwert 81 sind, am kleinsten ist, wird als kleinste verbleibende Amplitude 33 ausgewählt. Diejenige Amplitude eines Ereignisses 23, die unter den Amplituden, die kleiner als der Schwellenwert 81 sind, am größten ist, wird als größte verworfene Amplitude 34 ausgewählt.

Anstelle des ermittelten Minimums 51 und Maximums 52 können bei den in **Fig. 7** und **Fig. 8** dargestellten vereinfachten Verfahren auch jeweils der Mittelwert oder Median der kleinsten verbleibenden Amplituden 33 sowie der Mittelwert oder Median der größten verworfenen Amplituden 34 als oberer und unterer Schwellenwert 51, 52 herangezogen werden. Weiters können auch diejenigen kleinsten verbleibenden Amplituden 33 sowie diejenigen größten verworfenen Amplituden 34, die für ein mit Artefakten in Zeitbereichen 61 behaftetes Teilsignal 11 ermittelt worden sind, bei der Bestimmung des Minimums 51, des Maximums 52 oder des oberen oder unteren Schwellenwerts 51, 52 ausgeschlossen und nicht herangezogen werden.

Das beschriebene Verfahren kann adaptiv geführt werden. Mit jedem Schritt werden bei der Bestimmung der Schwellenwerte 51, 52 jeweils die kleinsten verbleibenden Amplituden 33 sowie die größten verworfenen Amplituden 34 mit berücksichtigt.

Die mit **Fig. 8** als alternativ dargestellten Vereinfachungen können auch als zusätzlicher Anhaltspunkt bei der Bestimmung des größten Abstands 27 zwischen jeweils zwei Amplituden herangezogen werden. Ist beispielsweise innerhalb eines Teilsignals 11 nicht eindeutig festzustellen, wo der größte Abstand 27 liegt und somit wo die Trennung zwischen verworfenen und gültigen Ereignissen 23 durchgeführt werden soll, beispielsweise weil es in der sortierten Folge von Amplituden mehrere in Frage kommende Sprünge zwischen jeweils zwei in der Sortierung benachbarten Ereignissen 23 und deren jeweils zugeordneten Amplituden gibt, kann bei der Auswahl der kleinsten verbleibenden Amplitude 33 mitberücksichtigt werden, bei welchen Amplitudenwerten die bisher ermittelten Differenzen von Amplituden ausgewählt wurden. Es kann beispielsweise jene Differenz ausgewählt und die zugehörigen Amplituden 23 als maximale verworfene und minimale verbleibende Amplitude 33, 34 gewählt werden, die am ehesten den bisher aufgefundenen Differenzen oder Amplituden entspricht.

So kann beispielsweise unter den infrage kommenden maximalen verworfenen und minimalen verbleibenden Amplituden 33, 34 diejenigen ausgewählt werden, die am besten mit dem Mittelwert 81 der bisher ermittelten maximalen verworfenen und minimalen verbleibenden Amplituden 33, 34 anderer Teilsignale 11 entspricht.

In jedem Teilsignal 11 kann parallel zur Bestimmung der gültigen Ereignisse 16 auch ein Qualitätsmaß Q bestimmt werden, welches angibt, wie zuverlässig die Detektion der gültigen Ereignisse 16 vorgenommen werden konnte. Beispielweise kann das Qualitätsmaß Q vom Abstand zwischen der größten verworfenen Amplitude 34 $amp_{R,max}$ und der kleinsten verbleibenden Amplitude 33, $amp_{E,min}$ abhängen.

Weiters kann die Regelmäßigkeit der gefundenen Ereignisse das Qualitätsmaß Q beeinflussen. So ist beispielsweise beim gesunden Herzen damit zu rechnen, dass Herzschläge in regelmäßigen Abständen auftreten. Extrem unregelmäßige Rhythmen sind entsprechend unwahrscheinlicher. Schließlich kann aus bekannten Normwerten vorhergesagt werden, welche Amplituden die Ereignisse 16 mit größter Wahrscheinlichkeit annehmen werden. Beim QRS-Komplex sind beispielsweise Amplituden zwischen 0.2 bis 3 mV zu erwarten. Größere oder kleinere Amplituden könnten auf schlechte Signalqualität des Biosignals 1 hinweisen. Das Qualitätsmaß Q in einem Teilsignal 11 kann aber auch von davon unabhängigen Maßen, beispielsweise vom Anteil flacher Signalstrecken, von der maximalen Steigung, von der Anzahl an Impulsen mit starken Flanken, etc. abhängen. Schließlich kann das Qualitätsmaß Q auch davon abhängen, wie sehr sich verschiedene Signalparameter - beispielsweise $amp_{R,max\ 3}$ 34 und $amp_{E,min}$ 33 - im Vergleich zu anderen Teilsignalen 11 verändert haben.

Für jedes Teilsignal 11 des Biosignals 1 kann ein Qualitätsmaß Q ermittelt werden, das angibt, wie hoch die Wahrscheinlichkeit des Auftretens eines Artefakts in diesem Teilsignal 11 ist. In dieser besonderen Ausführungsform der Erfindung werden pro Teilsignal 11 vier Teilmaße Q1, Q2, Q3, Q4 ermittelt, anschließend wird aus der Summe dieser Teilmaßen Q1, Q2, Q3, Q4 ein Qualitätsmaß Q nach der Formel Q = Q1 + Q2 + Q3 + Q4 bestimmt. Die Teilmaße Q1, Q2, Q3, Q4 können wie folge bestimmt werden:

$$Q1 = (amp_{R,max} - amp_{E,min}) / amp_{R,max}$$

Q2 = 1 / (Standardabweichung der zeitlichen Abstände zwischen den gültigen Ereignissen 16)

Q3 = 1, falls alle Amplituden der erkannten gültigen Ereignisse 16 zwischen 0,1 und 3 mV liegen; 0,1 sonst

Q4 = 1 **/ (abs** ($amp_{R,max}$ - **mean**($amp_{R,max}$ (t)) + **abs**($amp_{E,min}$ - **mean** ($amp_{E,min}$ (t)))

Die Funktionen **abs** bezeichnet den Betrag einer reellen Zahl, die Funktion **mean** bezeichnet den zeitlichen Mittelwert einer Anzahl von Funktionswerten. Wie bereits erwähnt, bezeichnet das Symbol $amp_{R,max}$ die größte verworfene Amplitude 34 und das Symbol $amp_{E,min}$ die kleinste verbleibende Amplitude 33 des jeweiligen Teilsignals 11.

Bereits bei der Bestimmung der kleinsten verbleibenden Amplitude 33 und der größten verworfenen Amplitude 34 können die bisher ermittelten kleinsten verbleibenden Amplituden 33, $amp_{E,min}$ und größten verworfenen Amplituden 34, $amp_{R,max}$ der bisherigen Teilsignale 11 verwendet werden, um die Menge möglicher Vergleiche von Amplituden von Ereignissen 23 einzuschränken (siehe **Fig. 7**). Bei der Suche nach der größten Differenz 27 zwischen Amplituden zweier aufeinander folgender Ereignisse 23 kann der Suchraum auch dadurch eingeschränkt werden, dass die Anzahl an Ereignissen 23 mindestens einen sinnvoll anzunehmenden Minimalwert aufweist, so kann etwa vorgegeben sein, dass bei einem EKG-Signal etwa 30 Ereignisse pro Minute Signallänge mindestens und 300 gültige Ereignisse 16 maximal vorhanden sind, dass die Amplitude von gültigen Ereignissen 16 zwischen zwei vorgegebenen Werten, beispielsweise bei einem EKG-Signal zwischen einem Maximalwert $amp_{max}$ von etwa 3.0 mV und einem Minimalwert $amp_{min}$, z.B. 0.2 mV, liegen.

Ein weiteres Vorgehen zur Ermittlung der kleinsten verbleibenden Amplitude 33 nutzt die Auswertung des Qualitätsmaßes Q für das jeweilige Teilsignal 11. Die infrage kommenden kleinsten verbleibenden Amplituden 33 werden vorab festgelegt, wobei gegebenenfalls die Amplituden von einigen Ereignissen 23 nach den voranstehend genannten Methoden vorab ausgeschlossen werden können. Die kleinste verbleibende Amplitude 33 wird jeweils auf den Amplitudenwert der infrage kommenden Ereignisse 23 gesetzt und anschließend wird der Qualitätswert Q mit der Maßgabe ausgewertet, dass dieser Amplitudenwert der kleinsten verbleibenden Amplitude 33 entspricht. Schließlich wird derjenige Amplitudenwert als kleinste verbleibende Amplitude 33 ermittelt, für den das Qualitätsmaß Q jeweils die höchste Qualität indiziert hat.

Falls das Qualitätsmaß Q innerhalb eines Teilsignals 11 einen Wert annimmt, der unter einem vordefinierten Schwellwert liegt, oder der sich deutlich von den Qualitätsmaßen Q anderer, insbesondere unmittelbar vorangehender oder unmittelbar nachfolgender, Teilsignale 11 signifikant unterscheidet, so können daraus unterschiedliche Maßnahmen abgeleitet werden. Insbesondere kann das Teilsignal 11 in Teilbereiche unterteilt werden, in denen dann separat nach Ereignissen 23 gesucht wird.

Als signifikant abweichend kann beispielsweise ein Qualitätsmaß gelten, wenn es um mehr als 20 % geringer ist als der Mittelwert der Qualitätsmaße der bisherigen Teilsignale 11.

Zur Abhilfe wird das Teilsignal 11 in Teilbereiche unterteilt, in denen dann separat nach Ereignissen 23 gesucht wird. Sollte sich nur in einem der Teilbereiche ein Artefakt befinden, d. h. das Qualitätsmaß Q nur in einem der Teilbereiche signifikant schlechter sein als in den übrigen Teilbereichen, so wird die Detektion von Ereignissen 23 in den verbleibenden Teilbereichen mit hoher Qualität Q möglich sein. Nur der Teilbereich mit dem Artefakt würde in diesem Fall keine qualitativ hochwertige Detektion ermöglichen und könnte allenfalls verworfen werden.

**Patentansprüche**

1.  Verfahren zur Detektion von Artefakten in einem Biosignal mit wiederholt auftretenden Ereignissen,

a) wobei das Biosignal (1) in eine Anzahl von Teilsignalen (11), insbesondere derselben Länge, unterteilt wird, und für jedes einzelne Teilsignal (11)

- nach einer Anzahl von Ereignissen (23) gesucht wird, und die Amplitude des Teilsignals (11) zum Zeitpunkt des Auftretens des Ereignisses (23) ermittelt wird,
- die so aufgefundenen Ereignisse (23) nach ihrer Amplitude sortiert werden, und
- nach vorgegebenen Kriterien die Amplitude eines Ereignisses (23) als kleinste verbleibende Amplitude ermittelt und ausgewählt wird und die nächst kleinere Amplitude des in der Sortierung nachfolgenden Ereignisses (23) als größte verworfene Amplitude ermittelt wird,

b) wobei zum Erkennen von Artefakten innerhalb eines Teilsignals (11) die kleinste verbleibende Amplitude eines Teilsignals und/oder die größte verworfene Amplitude eines Teilsignals (11) mit den kleinsten verbleibenden Amplituden der übrigen Teilsignale (11) und/oder mit den größten verworfenen Amplituden der übrigen Teilsignale (11) verglichen wird und

c) bei Vorliegen von durch diesen Vergleich ermittelten, einen vorgegebenen Schwellenwert überschreitenden Abweichungen festgestellt wird, dass das Teilsignal (11) Artefakte aufweist.

2. Verfahren nach Anspruch 1, **dadurch gekennzeichnet, dass** zur Bestimmung der kleinsten verbleibenden Amplitude eines Teilsignals (11) nach einem Ereignis (23) gesucht wird, dessen Amplitude zur jeweils nächst kleineren Amplitude unter allen Ereignissen (23) den größten absoluten oder relativen Abstand (27) hat, und diese Amplitude als kleinste verbleibende Amplitude (33) dem Teilsignal (11) zugeordnet wird und die nächstkleinere Amplitude dem Teilsignal als größte verworfene Amplitude (34) zugeordnet wird.

3. Verfahren nach Anspruch 1 oder 2, **dadurch gekennzeichnet, dass** zur Bestimmung der kleinsten verbleibenden Amplitude eines Teilsignals (11)

- für eine Anzahl von unmittelbar vorangehenden Teilsignalen (11) ein Schwellenwert (81) als, insbesondere arithmetischer, Mittelwert aller kleinsten verbleibenden Amplituden (33) und aller größten verworfenen Amplituden (34) mit oder ohne Gewichtung ermittelt wird,
- im jeweiligen Teilsignal (11) das Ereignis (23) mit der kleinsten diesen Schwellenwert (81) übersteigenden Amplitude ausgewählt und diese Amplitude als kleinste verbleibende Amplitude (33) für dieses Teilsignal (11) ermittelt wird, und
- die jeweils nächstkleinere Amplitude als größte verworfene Amplitude (34) für dieses Teilsignal (11) ermittelt wird.

4. Verfahren nach einem der vorangehenden Ansprüche, **dadurch gekennzeichnet, dass** zur Bestimmung der kleinsten verbleibenden Amplitude (33) eines Teilsignals (11) nach einer vorgegebenen Anzahl von Ereignissen (23) gesucht wird, deren Amplitude sich am stärksten von der nächstkleineren Amplitude unterscheidet, und dass unter diesen Ereignissen (23) und ihren zugehörigen Amplituden diejenige Amplitude als kleinste verbleibende Amplitude (33) des Teilsignals (11) ausgewählt wird, bei der ein aus dieser Amplitude und der nächstkleineren Amplitude nach vorgegebenen Vorgaben berechneter Wert, insbesondere deren Mittelwert, oder die Amplitude selbst oder die nächstkleinere Amplitude selbst, am nächsten bei einem vorgegebenen Schwellenwert (81), insbesondere beim Mittelwert der nach denselben Vorgaben jeweils in den übrigen Teilsignalen (11) berechneten Werte, liegt.

5. Verfahren nach einem der vorangehenden Ansprüche, **dadurch gekennzeichnet, dass** ein Qualitätsmaß (Q) für jedes Teilsignal (11) ermittelt wird, das eine oder mehrere der folgenden Eigenschaften aufweist:

a) der Unterschied zwischen der kleinsten verbleibenden Amplitude (33) und der größten verworfenen Amplitude (34) ist proportional zum Qualitätsmaß, wobei ein großer Unterschied eine gute Qualität des Teilsignals indiziert,
b) treten die gültigen Ereignisse (16) in regelmäßigen Abständen auf, so indiziert dies eine gute Qualität des Teilsignals (11),
c) liegen die Amplituden der gültigen Ereignisse (16) in einem vorgegebenen physiologisch erwartbaren Bereich auf, so indiziert dies eine gute Qualität des Teilsignals (11),
d) ähneln die kleinesten verbleibenden Amplituden (33) und/oder die größten verworfenen Amplituden (34) im jeweiligen Teilsignal (11) jenen einer Anzahl anderer Teilsignale (11) desselben Biosignals (1), so indiziert das eine gute Qualität des Teilsignals (11).

6. Verfahren nach Anspruch 5, **dadurch gekennzeichnet, dass** das Qualitätsmaß (Q) als gewichtete Summe oder als Produkt eines oder mehrerer der folgenden Qualitätsteilmaße (Q1, Q2, Q3, Q4) ermittelt wird:

  a) Q1 = (kleinste verbleibende Amplitude (33) - größte verworfene Amplitude (34)) / größte verbleibende Amplitude,
  b) Q2 = 1 / (Standardabweichung der zeitlichen Abstände aller aufeinanderfolgenden gültigen Ereignisse (16)),
  c) Q3 = 1, falls die Amplituden aller gültigen Ereignisse (16) zwischen 0.1 und 3 mV liegen, sonst 0.1,
  d) Q4 = 1 / (abs ($amp_{R,max}$- mean($amp_{R,max}$ (t)) + abs($amp_{E,min}$- mean ($amp_{E,min}$ (t))),
  wobei das Qualitätsmaß insbesondere gemäß der folgenden Formel ermittelt wird:

$$Q = Q1 * Q2 * Q3 * Q4 \qquad \text{oder} \qquad Q = Q1 + Q2 + Q3 + Q4$$

7. Verfahren nach einem der Ansprüche 5 oder 6, **dadurch gekennzeichnet, dass** zur Bestimmung der kleinsten verbleibenden Amplitude (33) innerhalb des Teilsignals (11) für eine Anzahl von Ereignissen (23), insbesondere für alle Ereignisse (23), das Qualitätsmaß (Q) unter der Annahme ermittelt wird, dass die dem jeweiligen Ereignis (23) zugehörende Amplitude die kleinste verbleibende Amplitude (33) ist,
und die kleinste verbleibende Amplitude (23) als demjenigen Ereignis zugeordnete Amplitude bestimmt und festgelegt wird, für das das jeweilige Teilsignal (11) einen Qualitätswert (Q) erzielt, der unter allen Ereignissen (23) die höchste Qualität indiziert.

8. Verfahren nach einem der Ansprüche 5 oder 6, **dadurch gekennzeichnet,**

  - **dass** die Bestimmung der kleinsten verbleibenden Amplitude (33) eines Teilsignals (11) nach einem der Ansprüche 3 oder 4 erfolgt,
  - **dass** für dieses Teilsignal (11) jeweils ein Qualitätswert (Q) nach einem der Ansprüche 5 oder 6 ermittelt wird, und
  - falls der Qualitätswert eines Teilsignals (11) eine schlechtere Qualität indiziert als ein vorgegebener Schwellenwert oder sich die Qualität um mehr als einen vordefinierten Schwellwert von jener einer vorgegebenen Auswahl anderer Teilsignale (11) unterscheidet, die kleinste verbleibende Amplitude (33) eines Teilsignals (11) gemäß einem Verfahren nach einem der Ansprüche 2 oder 7 bestimmt wird.

9. Verfahren nach einem der vorangehenden Ansprüche, **dadurch gekennzeichnet, dass** Teilsignale (11) dann als mit Artefakten behaftet angesehen werden, wenn in diesen Teilsignalen (11) entweder die minimale verbleibende Amplitude (33) kleiner ist als das Maximum (52) der bisher ermittelten, und gegebenenfalls gespeicherten, maximalen verworfenen Amplituden (34) oder die maximale verworfene Amplitude (34) größer ist als das Minimum (51) der bisher ermittelten, und gegebenenfalls gespeicherten, minimalen verbleibenden Amplituden (33).

10. Verfahren nach einem der Ansprüche 5 bis 8, **dadurch gekennzeichnet, dass** Teilsignale (11) verworfen und nicht zur Beurteilung nachfolgender Teilsignale (11) herangezogen, und insbesondere nicht abgespeichert, werden, wenn ihr Qualitätsmaß (Q) einen vorgegebenen Schwellenwert unterschreitet oder wenn sie als mit Artefakten behaftet detektiert worden sind.

11. Verfahren nach einem der vorangehenden Ansprüche, **dadurch gekennzeichnet, dass** die Teilsignale (11) im Biosignal (1) lückenlos und unmittelbar hintereinander liegen oder die Teilsignale (11) einander überlappen.

12. Verfahren nach einem der vorangehenden Ansprüche, **dadurch gekennzeichnet, dass** die Teilsignale (11) gleich lang sind und/oder die Startzeitpunkte aufeinanderfolgender oder überlappender Teilsignale (11) zeitlich gleich weit voneinander beabstandet sind.

13. Verfahren nach einem der vorangehenden Ansprüche, **dadurch gekennzeichnet, dass**, insbesondere zur Beschleunigung des Verfahrens, bei der Beurteilung eines Biosignals (1) mit zeitlich überlappenden Teilsignalen (11) die bereits berechneten Werte überlappender Teilsignale (11) zwischengespeichert werden und jeweils nur Werte für die hinzu gekommenen Zeitbereiche erneut berechnet und die im neuen Teilsignal (11) nicht mehr vorhandenen Zeitbereiche verworfen werden.

14. Verfahren nach einem der vorangehenden Ansprüche, **dadurch gekennzeichnet, dass**, insbesondere zur Beschleunigung des Verfahrens, innerhalb des aktuellen Teilsignals (11) nur Amplituden als größte verworfene Amplitude (34) in Betracht gezogen werden, die kleiner sind als ein aus einer Anzahl kleinster verbleibender Amplituden (33) anderer Teilsignale (11) berechneter Wert, insbesondere als deren Maximum oder deren Mittelwert, und/oder nur Amplituden als kleinste verbleibende Amplitude (33) in Betracht gezogen werden, die größer sind als ein aus einer Anzahl größter verworfener Amplituden (34) anderer Teilsignale (11) berechneter Wert, insbesondere als deren Minimum oder deren Mittelwert.

**Claims**

1. A process for detecting artifacts in a biosignal with repeating events,

   a) said biosignal (1) being subdivided into a number of sub-signals (11), particularly of the same length, and for each sub-signal (11)

   - a number of events (23) being searched for and the amplitude of each sub-signal (11) being determined at the point in time when the event (23) occurs,
   - the thus detected events (23) being sorted according to their amplitudes, and
   - the amplitude of one event (23) being determined and selected as the lowest remaining amplitude according to predefined criteria and the next lower amplitude of the event (23) next in order being determined as the highest discarded amplitude,

   b) the lowest remaining amplitude of a sub-signal and/or the highest discarded amplitude of a sub-signal (11) being compared to the lowest remaining amplitudes of the other sub-signals (11) and/or to the highest discarded amplitudes of the other sub-signals (11) to detect artifacts within said sub-signal (11), and
   c) if deviations exceeding a predetermined threshold are found in the course of the above comparison, said sub-signal (11) is determined to contain artifacts.

2. The process according to claim 1, **characterised in that**, in order to determine the lowest remaining amplitude of a sub-signal (11 )an event (23) whose amplitude has the greatest absolute or relative interval (27) of all events (23) to the respective next lower amplitude is searched for and this amplitude is assigned to the sub-signal (11) as the lowest remaining amplitude (33) and the next lower amplitude is assigned to the sub-signal as the highest discarded amplitude (34).

3. The process according to claim 1 or 2, **characterised in that** in order to determine the lowest remaining amplitude of a sub-signal (11)

   - a threshold value (81) for a number of immediately preceding sub-signals (11) is determined as, particularly arithmetic, mean value of all lowest remaining amplitudes (33) and all highest discarded amplitudes (34) with or without weighting,
   - in each sub-signal (11) the event (23) with the lowest amplitude exceeding this threshold value (81) is selected and determined as the lowest remaining amplitude (33) for the respective sub-signal (11), and
   - the next lower amplitude is determined as highest discarded amplitude (34) for the respective sub-signal (11).

4. The process according to any one of the preceding claims, **characterised in that** in order to determine the lowest remaining amplitude (33) of a sub-signal (11) a predefined number of events (23) whose amplitude differs most significantly from the next lower amplitude is searched for and
   **in that** among these events (23) and the associated amplitudes the amplitude for which a value, particularly a mean value, calculated based on said amplitude and the next lower amplitude according to predefined rules or said amplitude itself or the next lower amplitude itself is closest to a predefined threshold value (81), particularly to the mean value of the values calculated for the remaining sub-signals (11) according to the same rules, is selected as the lowest remaining amplitude (33) of the sub-signal (11).

5. The process according to any one of the preceding claims, **characterised in that** a quality measure (Q) is determined for each sub-signal (11), said quality measure having one or more of the following characteristics:

   a) the difference between the lowest remaining amplitude (33) and the highest discarded amplitude (34) is proportional to the quality measure, a great difference indicating that a sub-signal's quality is good,

b) if valid events (16) occur periodically, this is an indication that a sub-signal's (11) quality is good,
c) if the amplitudes of the valid events (16) are all within a predefined, physiologically expectable range, this is an indication that a sub-signal's (11) quality is good,
d) if the lowest remaining amplitudes (33) and/or the highest discarded amplitudes (34) in a sub-signal (11) are similar to those of a number of other sub-signals (11) of the same biosignal (1), this is an indication that a sub-signal's (11) quality is good.

6. The process according to claim 5, **characterised in that** the quality standard (Q) is determined as a weighted sum or as a product of one or more of the following quality sub-measure (Q1, Q2, Q3, Q4):

a) Q1 = (lowest remaining amplitude (33) - highest discarded amplitude (34))/highest remaining amplitude;
b) Q2 = 1 / (standard deviation of the time intervals of all consecutive valid events (16)),
c) Q3 = 1, if the amplitudes of all valid events (16) range between 0.1 and 3 mV, otherwise 0.1,
d) Q4 = $1/(\text{abs}(\text{amp}_{R,max} - \text{mean}(\text{amp}_{R,max}(t)) + \text{abs}(\text{amP}_{E,min} - \text{mean}(\text{amP}_{E,min}(t)))$,
the quality measure being particularly determined according to the following formula:

$$Q = Q1*Q2*Q3*Q4 \text{ or } Q = Q1 + Q2 + Q3 + Q4.$$

7. The process according to claim 5 or claim 6, **characterised in that** in order to determine the lowest remaining amplitude (33) within the sub-signal (11) for a number of events (23), particularly for all events (23), the quality measure (Q) is determined assuming that the amplitudes associated with the respective events (23) are the lowest remaining amplitudes (33),
and the lowest remaining amplitude (23) is determined and defined as amplitude associated to the event whose sub-signal (11) has a quality measure (Q) indicating the highest quality among all events (23).

8. The process according to claim 5 or claim 6, **characterised in that**

- the lowest remaining amplitude (33) of a sub-signal (11) is determined according to claim 3 or claim 4,
- a quality measure (Q) for said sub-signal (11) is determined according to claim 5 or claim 6, and
- if the quality measure of a sub-signal (11) indicates a quality which is lower than a predefined threshold value or if the quality differs from that of a predefined selection of other sub-signals (11) by more than a predefined threshold value, the lowest remaining amplitude (33) of a sub-signal (11) is determined by a process according to claim 2 or claim 7.

9. The process according to any one of the preceding claims, **characterised in that** sub-signals (11) are considered to comprise artifacts, if the lowest remaining amplitude (33) of said sub-signals (11) is lower than the maximum value of the highest discarded amplitudes (34) determined and optionally stored up to that point in time or if the highest discarded amplitude (34) is higher than the minimum value (51) of the lowest remaining amplitudes (33) determined and optionally stored up to that point in time.

10. The process according to any one of the claims 5 to 8, **characterised in that** sub-signals (11) are discarded and not used for the evaluation of subsequent sub-signals (11) and particularly not stored, if their quality measures (Q) are below a predefined threshold value or if they have been detected as comprising artifacts.

11. The process according to any one of the preceding claims, **characterised in that** the sub-signals (11) in the biosignal (1) immediately follow one another without any interruptions between them or overlap.

12. The process according to any one of the preceding claims, **characterised in that** the sub-signals (11) have the same length and/or the starting times of subsequent or overlapping sub-signals (11) are spaced apart by equal time periods.

13. The process according to any one of the preceding claims, **characterised in that**, when evaluating a biosignal (1) with overlapping sub-signals (11), calculated values of overlapping sub-signals (11) are stored and only values for new time intervals are newly calculated, discarding the time intervals which are no longer part of a new sub-signal (11), particularly to accelerate the process.

**14.** The process according to any one of the preceding claims, **characterised in that**, particularly to accelerate the process, only those amplitudes are taken into consideration as highest discarded amplitude (34) within a current sub-signal (11) which are lower than a value calculated based on a number of lowest remaining amplitudes (33) of other sub-signals (11), particularly lower than their maximum or mean value, and/or

only those amplitudes are taken into consideration as lowest remaining amplitude (33) which are higher than a value calculated based on a number of highest discarded amplitudes (34) of other sub-signals (11), particularly higher than their minimum or mean value.


**Revendications**

**1.** Procédé pour la détection d'artefacts dans un biosignal avec des événements répétitifs,

a) ledit biosignal (1) étant subdivisé dans un nombre de sous-signaux (11), notamment de la même durée, et pour chaque sous-signal (11)

- un nombre d'événements (23) étant recherché et l'amplitude de chaque sous-signal (11) étant déterminé à l'instant de leur occurrence,
- les événements (23) ainsi déterminés étant classés selon leur amplitudes et
- l'amplitude d'un événement (23) étant déterminée et sélectionnée comme la plus basse amplitude restante selon des critères définis et l'amplitude inférieure de l'événement (23) suivant étant déterminée comme la plus haute amplitude rejetée,

b) la plus basse amplitude restante d'un sous-signal et/ou la plus haute amplitude rejetée d'un sous-signal (11) étant comparée(s) aux plus basses amplitudes restantes d'autres sous-signaux (11) et/ou aux plus hautes amplitudes rejetées d'autres sous-signaux (11) pour la détection d'artefacts dans le sous-signal (11) respectif, et
c) quand la comparaison montre des déviations qui dépassent une valeur seuil définie, ledit sous-signal (11) contient des artefacts.

**2.** Procédé selon la revendication 1, **caractérisé en ce qu'**on recherche un événement (23) dont l'amplitude est écartée de l'amplitude inférieure respective par l'intervalle absolu ou relatif le plus important de tous événements (23) pour déterminer la plus basse amplitude restante d'un sous-signal (11) et cet amplitude est attribuée au sous-signal (11) comme la plus basse amplitude restante (11) et l'amplitude inférieure est attribuée au sous-signal (11) comme la plus haute amplitude rejetée (34).

**3.** Procédé selon la revendication 1 ou 2, **caractérisé en ce que** pour déterminer la plus basse amplitude restante d'un sous-signal (11)

- une valeur seuil (81) est déterminée pour un nombre de sous-signaux (11) immédiatement précédents comme moyenne, notamment arithmétique, de toutes les plus basses amplitudes restantes (33) et toutes les plus hautes amplitudes rejetées (34), sans ou avec pondération,
- l'événement (23) avec l'amplitude la plus basse dépassant cette valeur seuil (81) est sélectionné pour chaque sous-signal (11) et déterminé comme la plus basse amplitude restante (33) pour ledit sous-signal (11), et
- l'amplitude inférieure est déterminée comme la plus haute amplitude rejetée (34) pour ledit sous-signal (11).

**4.** Procédé selon une des revendications précédentes, **caractérisé en ce que**, pour déterminer la plus basse amplitude restante (33) d'un sous-signal (11), on recherche un nombre défini d'événements (23) dont les amplitudes sont les plus écartées des amplitudes inférieures respectives,
et **en ce que** parmi ces événements (23) et les amplitudes associées l'amplitude dont une valeur, notamment une moyenne, calculée par rapport à ladite amplitude et son amplitude inférieure selon des règles définis ou l'amplitude même ou son amplitude inférieure même est la plus proche de la valeur seuil (81), notamment de la moyenne des valeurs calculées pour les autres sous-signaux (11) selon les mêmes règles, est sélectionnée comme la plus basse amplitude restante (33) du sous-signal (11).

**5.** Procédé selon une des revendications précédentes, **caractérisé en ce qu'**une grandeur de qualité (Q) est déterminée pour chaque sous-signal (11), ladite grandeur de qualité ayant une ou plusieurs des propriétés suivantes :

a) la différence entre la plus basse amplitude restante (33) et la plus haute amplitude rejetée (34) est proportionnelle à la grandeur de qualité, une différence significative indiquant un sous-signal de bonne qualité,
b) si des événements valables (16) ont lieu périodiquement, c'est une indication d'un sous-signal (11) de bonne qualité,
c) si les amplitudes des événements valables (16) se trouvent tous dans un écart défini et physiologiquement prévisible,c'est une indication d'un sous-signal (11) de bonne qualité,
d) si les plus basses amplitudes restantes (33) et/ou les plus hautes amplitudes rejetées (34) d'un sous-signal (11) sont similaires à celles d'un nombre d'autres sous-signaux (11) du même biosignal (11), c'est une indication d'un sous-signal (11) de bonne qualité.

6. Procédé selon la revendication 5, **caractérisé en ce que** la grandeur de qualité (Q) est déterminé comme somme pondérée ou produit d'une ou plusieurs sous-grandeurs de qualité (Q1, Q2, Q3, Q4):

a) Q1 = (la plus basse amplitude restante (33) - la plus haute amplitude rejetée (34))/la plus haute amplitude restante;
b) Q2 = 1 / (écart-type des intervalles de temps de tous événements valables (16) consécutifs),
c) Q3 = 1 si les amplitudes de tous événements valables (16) varient entre 0,1 et 3 mV, sinon : 0,1,
d) Q4 = $1/(\text{abs}(\text{amp}_{R,max}- \text{moyenne}(\text{amp}_{R,max}(t)) + \text{abs}(\text{amp}_{E,min} - \text{moyenne}(\text{amp}_{E,min}(t)))$,
la grandeur de qualité étant notamment déterminée selon l'une des formules suivantes :

$$Q = Q1{*}Q2{*}Q3{*}Q4 \text{ or } Q = Q1 + Q2 + Q3 + Q4.$$

7. Procédé selon la revendication 5 ou 6, **caractérisé en ce que**, pour déterminer la plus basse amplitude restante (33) d'un sous-signal (11) pour un nombre d'événements (23), notamment pour tous les événements (23), la grandeur de qualité (Q) est déterminée en supposant que l'amplitude associée à chaque événement (23) soit la plus basse amplitude restante (33),
et la plus basse amplitude restante (23) est déterminée et définie comme l'amplitude associée à l'événement dont le sous-signal (11) a une grandeur de qualité (Q) indiquant la meilleure qualité de tous événements (23).

8. Procédé selon la revendication 5 ou 6, **caractérisé en ce que**

- la plus basse amplitude restante (33) d'un sous-signal (11) est déterminée selon la revendication 3 ou 4,
- une grandeur de qualité (Q) pour ledit sous-signal (11) est déterminée selon la revendication 5 ou 6,
- si la grandeur de qualité d'un sous-signal (11) indique une qualité inférieure à une value seuil définie ou si la qualité diffère d'une sélection définie d'autres sous-signaux (11) par une valeur dépassant une valeur seuil définie, la plus basse amplitude restante (33) d'un sous-signal (11) est déterminée selon un procédé selon la revendication 2 ou 7.

9. Procédé selon une des revendications précédentes, **caractérisé en ce que** la présence d'artefacts dans des sous-signaux (11) est assumée si la plus basse amplitude restante (33) desdits sous-signaux (11) est plus basse que la valeur maximale des plus hautes amplitudes rejetées (34) déterminée et éventuellement enregistrée jusqu'à cet instant ou si la plus haute amplitude rejetée (34) est plus haute que la valeur minimale (51) des plus basses amplitudes restantes (33) déterminée et éventuellement enregistrée jusqu'à cet instant.

10. Procédé selon une des revendications 5 à 8, **caractérisé en ce que** les sous-signaux (11) sont rejetés et ne sont pas utilisés pour l'évaluation de sous-signaux (11) suivants et ne sont notamment pas enregistrés, si leur grandeurs de qualité (Q) restent inférieures à une valeur seuil définie ou si des artefacts ont été détectés dans ces sous-signaux (11).

11. Procédé selon une des revendications précédentes, **caractérisé en ce que** les sous-signaux (11) du biosignal (1) s'enchaînent de manière continue ou se chevauchent.

12. Procédé selon une des revendications précédentes, **caractérisé en ce que** les sous-signaux (11) sont de la même durée et/ou les débuts des sous-signaux (11) subséquents ou chevauchants sont écartés par des intervalles égaux.

13. Procédé selon une des revendications précédentes, **caractérisé en ce que** les valeurs calculées pour des sous-signaux (11) chevauchants pour l'évaluation d'un biosignal (1) avec des sous-signaux (11) chevauchants sont enregistrées et seulement les valeurs des intervalles nouveaux sont calculées de nouveau, les intervalles qui ne sont plus part du nouveau sous-signal (11) étant rejetés, notamment pour accélérer le procédé.

14. Procédé selon une des revendications précédentes, **caractérisé en ce que**, notamment pour accélérer le procédé, seulement ces amplitudes d'un sous-signal (11) actuel qui sont plus basses qu'une valeur calculée sur la base d'un nombre des plus basses amplitudes restantes (33) d'autres sous-signaux (11), notamment plus basses que leur valeur maximale ou moyenne, sont considérés comme la plus haute amplitude rejetée (34), et/ou
seulement ces amplitudes d'un sous-signal (11) actuel qui sont plus hautes qu'une valeur calculée sur la base d'un nombre des plus hautes amplitudes rejetées (34) d'autres sous-signaux (11), notamment plus hautes que leur valeur minimale ou moyenne, sont considérés comme la plus basse amplitude restante (33).

Fig 1

Fig. 2

Fig.3

Fig. 4

Fig. 5

Fig. 6

amp_max

27

34

33

amp_min

n_min

n_max

51

52

Fig. 7

41

51

81

42

52

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- AT 504167